# EUROPEAN PATENT SPECIFICATION

(11) **EP 2 627 198 B1**
(45) Date of publication and mention of the grant of the patent: **02.09.2015**
(21) Application number: 11768049.6
(22) Date of filing: 14.10.2011
(51) Int. Cl.: A23L 1/30, A61K 35/74, A61K 39/39

(54) **IMMUNOADJUVANT**
IMMUNADJUVANS
ADJUVANT IMMUNITAIRE

(30) Priority: 15.10.2010 EP 10187690
(43) Date of publication of application: 21.08.2013
(73) Proprietor: Chr. Hansen A/S, 2970 Hoersholm (DK)
(72) Inventor: ESKESEN, Dorte, 2840 Holte (DK)
(86) International application number: PCT/EP2011/068008
(87) International publication number: WO 2012/049301

(56) References cited:
- WO-A1-01/89541
- WO-A1-2006/007526
- WO-A2-2008/042101
- BOGE T ET AL: "A probiotic fermented dairy drink improves antibody response to influenza vaccination in the elderly in two randomised controlled trials", VACCINE, ELSEVIER LTD, GB, vol. 27, no. 41, 18 September 2009 (2009-09-18), pages 5677-5684, XP026502247, ISSN: 0264-410X, DOI: DOI:10.1016/J.VACCINE.2009.06.094 [retrieved on 2009-07-16]
- NAMBA KAZUYOSHI ET AL: "Effects of Bifidobacterium longum BB536 Administration on Influenza Infection, Influenza Vaccine Antibody Titer, and Cell-Mediated Immunity in the Elderly", BIOSCIENCE BIOTECHNOLOGY AND BIOCHEMISTRY, vol. 74, no. 5, May 2010 (2010-05), pages 939-945, XP002628361,
- OLIVARES ET AL: "Oral intake of Lactobacillus fermentum CECT5716 enhances the effects of influenza vaccination", NUTRITION, ELSEVIER INC, US, vol. 23, no. 3, 12 March 2007 (2007-03-12) , pages 254-260, XP005922809, ISSN: 0899-9007, DOI: DOI:10.1016/J.NUT.2007.01.004
- FRENCH P W ET AL: "Use of probiotic bacteria as an adjuvant for an influenza vaccine", INTERNATIONAL JOURNAL OF PROBIOTICS AND PREBIOTICS 2009 NEW CENTURY HEALTH PUBLISHERS, LLC USA, vol. 4, no. 3, August 2009 (2009-08), pages 175-180, XP009145972, ISSN: 1555-1431
- FUKUSHIMA Y ET AL: "Effect of a probiotic formula on intestinal immunoglobulin A production in healthy children", INTERNATIONAL JOURNAL OF FOOD MICROBIOLOGY, ELSEVIER SCIENCE PUBLISHERS, AMSTERDAM, NL, vol. 42, no. 1-2, 30 June 1998 (1998-06-30), pages 39-44, XP002521672, ISSN: 0168-1605, DOI: DOI:10.1016/S0168-1605(98)00056-7
- DE MORENO DE LEBLANC A ET AL: "Oral administration of L. casei CRL 431 increases immunity in bronchus and mammary glands", EUROPEAN JOURNAL OF INFLAMMATION, BIOMED CENTRAL, IT, vol. 3, no. 1, 1 January 2005 (2005-01-01) , pages 25-28, XP009109241, ISSN: 1721-727X
- AGUERO ET AL: "Beneficial immunomodulatory activity of Lactobacillus casei in malnourished mice pneumonia: effect on inflammation and coagulation", NUTRITION, ELSEVIER INC, US, vol. 22, no. 7-8, 1 July 2006 (2006-07-01) , pages 810-819, XP005526324, ISSN: 0899-9007, DOI: DOI:10.1016/J.NUT.2006.03.013
- HORI T ET AL: "EFFECT OF INTRANASAL ADMINISTRATION OF LACTOBACILLUS CASEI SHIROTA ON INFLUENZA VIRUS INFECTION OF UPPER RESPIRATORY TRACT IN MICE", CLINICAL AND DIAGNOSTIC LABORATORY IMMUNOLOGY, AMERICAN SOCIETY FOR MICROBIOLOGY, US, vol. 8, no. 3, 1 May 2001 (2001-05-01), pages 593-597, XP001016238, ISSN: 1071-412X, DOI: DOI:10.1128/CDLI.8.3.593-597.2001
- YASUI HISAKO ET AL: "Protection against influenza virus infection of mice fed Bifidobacterium breve YIT4064", CLINICAL AND DIAGNOSTIC LABORATORY IMMUNOLOGY, AMERICAN SOCIETY FOR MICROBIOLOGY, US, vol. 6, no. 2, 1 March 1999 (1999-03-01), pages 186-192, XP002288967, ISSN: 1071-412X
- DE VRESE M ET AL: "Probiotic bacteria stimulate virus-specific neutralizing antibodies following a booster polio vaccination", EUROPEAN JOURNAL OF NUTRITION, STEINKOPFF-VERLAG, DA, vol. 44, no. 7, 1 October 2005 (2005-10-01), pages 406-413, XP019383082, ISSN: 1436-6215, DOI: DOI:10.1007/S00394-004-0541-8

## Description

### Technical field

The present invention relates to the use of lactic acid bacteria for amplifying the specific immune response against an infectious agent.

### Background

According to the definition of the Food Agricultural Organization (FAO) and the World Health Organization (WHO), probiotics are live bacteria that offer a health benefit to the host when administered in adequate amounts (FAO/WHO 2001). Probiotics have shown a beneficial effect on different conditions such as lactose malabsorption, acute diarrhea, antibiotic-associated diarrhea, allergies, and inflammatory bowel disease (Goldin and Gorbach 2008).

Probiotic bacteria are generally found among the genera Lactobacillus, Streptococcus, Lactococcus and Bifidobacterium. A number of strains from these genera are commonly used for making cheese, yoghurt, and other dairy products.

Probiotics are thought to intervene in particular at the level of the intestinal flora by impeding the development of pathogenic microorganisms, and/or by acting more directly on the immune system. It has, for example, been observed that ingestion of probiotic bacteria or fermented foods, such as yogurt, comprising these bacteria, leads to a decrease in pathogenic bacteria; in terms of the immune system, various effects have been reported: an activation of the cells involved in the specific or nonspecific immune response, such as lymphocytes and macrophages, an increase in the level of immunoglobulins and in particular of IgA; an increase in the level of immune system-activating cytokines, etc. (for review, cf. for example MEYDANI and HA (Am J Clin Nutr, 71, 861-7217, 2000).

Overall, studies carried out on various probiotic lactic acid bacteria tend to conclude that some species, or at least some strains of these species, have immunostimulatory properties. Several studies carried out in humans and animals suggest that bacteria of the species L. casei have a beneficial effect on the health, and in particular a positive effect on the immune system.

### L. casei studies in mice

It has been shown in mice that ingestion of fermented milk containing the L. casei strain DN-114 001 increases resistance to infection with Salmonella typhimurium [PAUBERT-BRAQUET et al. Int J Immunother, 4:153, (1995)]; activation of macrophages and an increase in circulating IgAs have been observed simultaneously.

Malnourished mice that were fed *Lactobacillus paracasei* ssp. *paracasei* (*L. casei* 431^{®}) developed an improved resistance against pneumococcal respiratory infection (Villena et al. 2005). This was shown by a more effective pathogen clearance from the blood and significantly less lung damage as compared to the control group. In addition, the number of leukocytes and neutrophils was increased as well as the level of antipneumococcal IgA (Villena et al. 2005).

### L. casei studies in humans

In healthy adults, supplementation with chemically acidified milk containing *Lactobacillus rhamnosus* LGG^{®} (LGG^{®}) or *L. casei* 431^{®} resulted in increased circulating neutralizing antibody titres in a study where the subjects were vaccinated orally against polio at day eight. In particular, poliovirus-specific immunoglobulin A (IgA), IgG and IgM were increased (de Vrese et al. 2005). The authors concluded that probiotics induce an immunological response that may provide enhanced systemic protection of cells from virus infections by increasing production of virus neutralizing antibodies (de Vrese et al. 2005). The results reported in the article show that although relevant and indicating in the same direction, the differences were not in all cases significant. The only statistically significant result with regard to *L*. *casei* 431^{®} was that the effect on serotype-2-specific IgM was statistically significant.

### Studies with BB-12^{®} in children show increased fecal IgA

Several studies in children have shown immune modulating properties of the probiotic strain *Bifidobacterium animalis* ssp. lactis, BB-12^{®} (BB-12^{®}). The administration of BB-12^{®} in a follow-up formula to seven children (15 to 31 months old) who had completed the oral polio vaccination by 12 months of age significantly increased fecal concentrations of total IgA and anti-poliovirus IgA, which may contribute to enhanced mucosal resistance against gastrointestinal infections (Fukushima et al. 1998). Consumption of a combination of LGG^{®} and BB-12^{®} by formula-fed infants during the first year of life resulted in an increase of cow milk-specific IgA secreting cells at the time of introduction of cow's milk in the infant's diet (Rautava et al. 2006). In preterm infants, oral application of BB-12^{®} for 21 days resulted in increased fecal IgA as compared to the control group. In addition, body weight, fecal concentrations of acetate and lactate were higher, the latter finding indicating an improved lactose digestion. Fecal calprotectin was decreased in the probiotic group, demonstrating improved mucosal immunity and attenuation of inflammatory responses to dietary and bacterial antigens (Mohan et al. 2008).

### Influenza vaccine model in other L. casei strains than L. casei 431^{®}

Results from earlier studies employing the influenza vaccine model have been described for other probiotic strains. In a pilot study, consumption for 7 weeks of a fermented dairy product containing the probiotic strain *Lactobacillus casei* DN-114 001 in 86 elderly subjects over 70 years of age (n=86) resulted in an improved immune response to influenza vaccination. Seroprotection and seroconversion rates against specific influenza strains were consistently higher in the probiotic group than in the control group. In addition, influenza-specific antibody titres increased after vaccination, being consistently higher in the probiotic product group compared to the control group. No differences between groups were, however, statistically significant (Boge et al. 2009). Subsequently, a confirmatory study was performed in 222 elderly volunteers (mean age 85 years) who consumed either a fermented dairy drink containing *Lactobacillus casei* DN-114 001 or a non-fermented control dairy product for 13 weeks: Also in this study, specific antibody titres were consistently higher in the probiotic product group than in the control group following vaccination. The B strain antibody titre and seroconversion were significantly higher in the probiotic group versus control group over the entire product consumption period. Significant differences in seroconversion rate between the groups by intention to treat (ITT) analysis were still observed 5 months after vaccination for the B and H3N2 strains. (Boge et al. 2009).

In healthy adult volunteers (n=50), oral intake of a capsule containing *Lactobacillus fermentum* CECT5716 significantly increased antigen-specific IgA and total IgM after influenza vaccination and resulted in a higher concentration of these antibodies in the probiotic group compared to the control group 2 weeks after vaccination. In addition, the proportion of natural killer (NK) cells increased 2 weeks after the influenza vaccination in the probiotic group. Finally, the incidence of influenza-like illness (ILI) during the 5-month period after vaccination was lower in the group consuming probiotics compared to the control group with a significant difference between groups at 5 months (in February) (Olivares et al. 2007).

Namba et al. (2010) Effects of Bifidobacterium longum BB536 Administration on Influenza Infection, Influenza Vaccine Antibody Titer, and Cell-Mediated Immunity in the Elderly, Bioscience, Biotechnology and Biochemistry, 74(5), 939-945, reveal that *Bifidobacterium longum* (BB536) reduces the incidence of influenza and fever in elderly individuals to whom influenza vaccine was administered.

Despite such studies, because health benefit of probiotics are strain-dependent, the functional effects demonstrated for one probiotic strain cannot necessarily be extrapolated to other strains (Food and Agriculture Organization of the United Nations and World Health Organization (2002). Guidelines for the Evaluation of Probiotics in Food. Report of a Joint FAO/WHO Working Group on Drafting Guidelines for the Evaluation of Probiotics in Food. London/Ontario: FAO/WHO and Rijkers GT, Bengmark S, Enck P, et al (2010) Guidance for substantiating the evidence for beneficial effects of probiotics: current status and recommendations for future research J Nutr 140, 671 S-676S). As human data on the immune-modulating effect of *Bifidobacterium animalis* ssp. *lactis* (BB-12^{®}) and *Lactobacillus paracasei* ssp. *paracasei* (*L. casei* 431^{®)} are very sparse, it was considered important to further investigate the effects of these two probiotic strains in a controlled, adequately powered trial using the challenge of influenza vaccination as there is still a need to develop further, more efficient, technologies for eliciting an efficient immune response.

### Summary of the invention

The inventor has shown that administration of L. casei 431 or BB-12 in conjuction with an influenza vaccine gives rise to a stronger immune response than that seen with vaccine only. The invention thus relates to a probiotic bacterium such as L. casei 431 or BB-12 for use in a method of boosting the immune response in conjunction with an influenza vaccine according to the appended claims.

### Figures

- **Figure 1**: **Vaccine-specific plasma IgG at Baseline and Day 42 by Study Group**
- **Figure 2**: **Vaccine-specific plasma IgG1 at Baseline and Day 42 by Study Group**
- **Figure 3**: **Vaccine-specific plasma IgG3 at Baseline and Day 42 by Study Group**
- **Figure 4**: **Vaccine-specific salivary IgG at Baseline and Day 42 by Study Group**
- **Figure 5**: **Vaccine-specific salivary IgA at Baseline and Day 42 by Study Group**
- **Figure 6**: **Vaccine-specific salivary IgM at Baseline and Day 42 by Study Group**

### Detailed description of the invention

To examine the effect of probiotics on the immune response, the present study utilized an influenza vaccine model for the following reasons:
- The natural exposure to pathogens is highly uncontrolled and unpredictable, and by using a vaccine model, the natural exposure is exchanged with a controlled exposure to killed or attenuated micro-organisms (vaccine). A vaccine triggers an *in vivo* immune response which is indicative of the ability of the immune system to elicit an antigen-specific response to pathogens (Albers et al. 2005). Response to vaccination is a widely used marker of immune function which provides high-quality information on the effect of nutrients on protective *in vivo* immune responsiveness (Albers et al. 2005). The vaccine model is a well studied model and results are therefore easy to compare with results from previous studies. The influenza vaccine is used every year and is therefore an easily accessible model. The model can be used in a vast number of people making it easier to recruit an adequate number of study participants.

The inventors have investigated whether *Lactobacillus paracasei* ssp. *paracasei* or *Bifidobacterium animalis* ssp. *lactis* also have an action on adaptive immunity, which, unlike innate immunity, results in a specific immune response against a given pathogenic agent.

For this purpose, the current study was designed to investigate the immune modulating properties of *Lactobacillus paracasei* ssp. *paracasei (L. casei* 431^{®}, previously deposited at the American Type Culture Collection with the deposit number ATCC55544) and *Bifidobacterium animalis* ssp. *lactis* (BB-12^{®}, previously deposited at the Deutsche Sammlung von Mikroorganismen und Zellkulturen GmbH with the deposit number DSM15954) in an influenza vaccination model. Both of these strains are commercially available from Chr. Hansen A/S, Hørsholm, Denmark. The aim with the present studies was to determine the effect of consumption of a milk-based drink containing *L. casei* 431^{®} or supplementation with a capsule containing BB-12^{®} on the specific immune response to the vaccine and the general immune response as compared to a placebo drink or placebo capsule, respectively.

The results herein show that the consumption of a minidrink containing *Lactobacillus paracasei* ssp. *paracasei,* L. casei 431^{®} or supplementation with a capsule containing the probiotic strain *Bifidobacterium animalis* ssp. *lactis,* BB-12^{®} resulted in an increased *in vivo* immune response to an influenza vaccine. The elicitation and strengthening of multiple and complementary effector mechanisms demonstrated in this study are considered to be associated with the best protection against mucosally-transmitted pathogens, such as the influenza virus. Thus, since both influenza-specific and total antibodies were increased in the L.casei 431^{®} and BB-12^{®} product groups as compared to the corresponding placebo groups, these results imply the beneficial effect of probiotics on both mucosal and systemic immunity.

The term "immune boosting" is meant to include situations in which the concentration of vaccine specific antibodies such as IgG, IgG1, and IgG3 were significantly increased in treatment groups compared with corresponding placebo treatment groups. Such immune boosting may be affective in preventing or treating e.g. diseases which are caused by trans-mucosal viruses, e.g. influenza, common cold, etc.

The pathogens concerned are especially bacteria or viruses; among the latter, mention will be made, for example, of rhinoviruses, respiratory syncytial virus (RSV), and myxoviruses (orthomyxoviruses such as the influenza viruses (influenza type A, B or C), or para-myxoviruses).

In the context of the implementation of the present invention, the strains *Lactobacillus paracasei* ssp. *paracasei (L. casei* 431^{®}, ATCC55544) and *Bifidobacterium animalis* ssp. *lactis* (BB-12^{®}, DSM15954) may be used alone or in combination with other lactic acid bacteria of other species. Advantageously, it may be used in combination with yoghurt ferments, namely *Lactobacillus bulgaricus* and *Streptococcus thermophilus.*

The strains may be used in the form of whole bacteria, which may or may not be live, and also in the form of a bacterial lysate, or in the form of bacterial fractions.

Preferably, a composition prepared in the context of a use in accordance with the invention contains at least 10⁵, preferably at least 10⁶, generally between 1x10⁸ and 1.5x10⁹, cells per ml.

When the strains are used in combination with yoghurt ferments, said composition also advantageously comprises at least 10⁷, preferably between 2x10⁸ and 1x10⁹, *S. thermophilus* cells per ml, and at least 5x10⁵, and preferably between 4x10⁶ and 2x10⁷, *L. bulgaricus* cells per ml.

The strains most particularly suitable for use in the present invention are the *Lactobacillus paracasei* ssp. *paracasei* (*L. casei* 431^{®}, ATCC55544) and *Bifidobacterium animalis* ssp. *lactis* (BB-12^{®}, DSM15954) strains. These strains are probiotic strains.

Compositions prepared in accordance with the invention may be administered in the form of foods or of food supplements. They may, for example, be dairy products, and in particular fermented dairy products comprising at least said strain, optionally combined with other lactic acid bacteria, for example with yogurt ferments.

Compositions prepared in accordance with the present invention can be used in the context of the prevention and treatment of pathological conditions of infectious origin, and in particular of viral origin, and in particular of the flu. Preferably, in order to obtain an optimal effect, they will be administered for at least one week, and advantageously for at least 10 days, in an amount corresponding to the absorption of at least 10⁷, preferably at least 10⁸, generally between 10⁹ and 10¹², *L. casei* 431^{®} or BB-12^{®} cells.

Administration of the probiotic cells may be simultaneously with the vaccine, but it is preferred to administer the probiotic cells prior to administration of the vaccine, such as at least one, two, or three weeks prior to vaccination. The probiotic is preferably administered on a regular basis, such as once or twice daily. The probiotic may also be administered after vaccination, such as during a period of one, two, three, or four weeks after the vaccination. In a preferred embodiment, administration of the probiotic is made two weeks prior to vaccination and continued until four weeks after vaccination.

The invention thus provides a treatment schedule, wherein a probiotic bacteria such as the above mentioned strains are administered in conjunction with a vaccine, in doses and time schedules as described above.

The invention thus provides a probiotic bacterial strain with the deposit number ATCC55544 or DSM15954 for use as an immune boosting composition, wherein the composition is administered between 1 day and 20 days, or about 10 days, prior to vaccination.

In one embodiment, the invention provides a probiotic bacterial strain with the deposit number ATCC55544 or DSM15954 for use as an immune boosting composition, wherein the composition is administered during 6 weeks.

In a further embodiment, the invention provides a probiotic bacterial strain with the deposit number ATCC55544 or DSM15954 for use according to claim 1 or 2, wherein the composition is a milk-based drink or a capsule.

The present invention will be more clearly understood with the aid of the further description which will follow, which refers to non-limiting examples illustrating the properties of a *Lactobacillus casei* or *Bifidobacterium* strain for reinforcing the specific response to microbial antigens.

### Examples

### EXAMPLE 1

### Overall Study Design and Plan-Description

This was a randomized, double-blind, placebo-controlled parallel-group study assessing the effect of two probiotic strains on immunity in a vaccination model. The study included a screening phase of at least 2 weeks duration, a supplementation phase of 6 weeks duration during which vaccination was performed (after 2 weeks of supplementation), and a follow-up period of 10 weeks duration with no supplementation of probiotics. Blood and saliva samples for analyses of efficacy parameters were collected just before supplementation (Day 0) and after 6 weeks of supplementation (Day 42). Information regarding symptoms of Upper Respiratory Tract Infections/Symptoms (URTI) and Influenza-Like Illness (ILI), and infections was collected ongoing during the supplementation phase and again after 10 weeks of follow-up.

### The conditions for this study are as follows:

### Disposition of Subjects

A total of 221 subjects were eligible to participate in the study and were randomized to one of the four treatment groups. Fifty-six subjects were assigned to the placebo drink group, 59 to the L. casei 431^{®} drink group, 52 to the placebo capsule group and 54 to the BB-12^{®} capsule group. In total, 10 subjects dropped out during the study of which only one subject received the influenza vaccine.

The four treatment arms were a drink containing the probiotic strain *Lactobacillus paracasei* ssp. *paracasei*, L. casei 431^{®} (L. casei 431 ^{®}) or a placebo drink, a capsule containing the probiotic strain *Bifidobacterium animalis* ssp. *lactis*, BB-12^{®} (BB-12^{®}) or a placebo capsule. Placebo products were similar to the active product, however, without the active ingredient, and the subjects, the investigators, and the site staff were unaware of which product the subject consumed, only if it was a drink or a capsule.

### 1.1.1. Screening (Day -14 - 0)

At screening each potential subject was given a complete explanation of the purpose of the study and details of the treatments and assessments. The subject's eligibility for the study was assessed by collecting demographic information, including dietary habits and health history.

In addition, subjects were instructed in which fermented products they were not allowed to consume from the screening visit to the end of the study.

Screening could take place by phone. If the subjects were screened at the clinical centre and found eligible, V2 procedures were conducted at the same day.

### 1.1.2. Randomization Visit (Visit 2, Day 0, Baseline)

At V2 subjects signed the Informed Consent document. In addition, the following procedures and assessments were performed:
- Eligibility assessments (inclusion/exclusion criteria)
- Medical history
- Fasting blood samples for analyses of immunological markers
- Saliva samples for analyses of immunoglobulins
- Clinical examination including height and weight, vital signs (heart rate, blood pressure, temperature and respiratory rate), and infection status
- Recording of concomitant medication
- Randomization of eligible subjects
- Dispensing of all capsules necessary for the supplementation period or part of the drinks which were distributed in batches because of the expiry date
- Instruction on which fermented products the subjects were not allowed to consume during the study

Subjects were provided with a diary to collect the following information:
- Weekly recording of incidence, duration and severity on a defined list of cold and flu-like symptoms
- Recording of symptoms of Influenza-Like Ilness (ILI) every 4 weeks based on the symptom diary
- Recording of adverse effects (AEs) during the entire study
- Recording of missed doses of investigational product during the entire study Subjects started consuming the investigational product on day 0.

### 1.1.3. Vaccination Visit (Visit 3, Day 14)

At V3, subjects were given an intramuscular shot of the influenza vaccine specific for the virus involved in the 2008/2009 epidemic (Fluad^{®}, Novartis Vaccines and Diagnostics, Siena, Italy). In addition, a clinical examination of infection status was performed and AEs were assessed. Subjects continued with consumption of the investigational products for the next 4 weeks.

### 1.1.4. Evaluation Visit (Visit 4, Day 42)

At V4, the following procedures were performed:
- Fasting blood samples for analyses of immunological markers
- Saliva samples for analyses of immunoglobulins
- Clinical examination including height and weight, vital signs (heart rate, blood pressure, temperature and respiratory rate), and infection status
- AE assessment
- Recording of concomitant medication

### 1.1.5. Follow-up Visit (Visit 5, Day 112)

At V5, the following procedures were to be performed:
- Clinical examination including height and weight, vital signs (heart rate, blood pressure, temperature and respiratory rate), and infection status
- Adverse Events assessment
- Recording of concomitant medication

In the study protocol, this last visit was included to check for infection status and collect information about symptoms of Upper Respiratory Tract Infections/Symptoms (URTI) and Influenza-Like Illness (ILI). However, since the visit took place in August 2009 and the chance of finding these symptoms was minimal, it was decided to perform the last visit by phone, and question the subjects about influenza symptoms, AEs and use of concomitant medication.

### EXAMPLE 2

### Interventions Administered

Two groups were provided with either the placebo or investigational drinks (see Table 1). The drinks delivered approximately 57 kcal per 100 ml. The drinks were produced in five different batches during the study, and sent from Chr. Hansen to the site. The drinks were dispensed to subjects in smaller batches due to the limited shelf life of the drinks (4 weeks from date of production). At Visit 2 subjects received part of the bottles and returned twice to the site during the study to receive new batches of drinks.

The label on each drink contained the following information: 'IMPRESS-acidified milk with or without probiotics', 'For clinical use only', subject number, 'To be shaken and consumed together with lunch', 'Contains 110 mL', expiry date, storage conditions, Sponsor name, Trial code, and Investigator name.

**Table 1 Description of drinks for the IMPRESS-study**

| | **Acidified milk drink, placebo (Chr. Hansen A/S, Denmark)** | **Acidified milk drink, probiotic (Chr. Hansen A/S, Denmark)** |
|---|---|---|
| **Brief description** | Low fat milk acidified with GDL (glucono delta lacton), added sucrose and peach flavor | Low fat milk acidified with GDL, added sucrose, peach flavor and L. casei 431 ^{®} |
| **Ingredients** | Milk, water, sugar, GDL, pectin, flavor | Milk, water, sugar, GDL, pectin, flavor and probiotic culture (L. casei 431^{®}), minimum 1 x 10⁹ Colony Forming Units (CFU)/drink |
| **Supplied as** | Bottle with 110 ml Acidified Milk drink | Bottle with 110 ml Acidified Milk drink |
| **Production dates** | 03 Feb 2009; 24 Feb 2009; 17 Mar 2009; 31 Mar 2009; 21 Apr 2009 | 03 Feb 2009; 24 Feb 2009; 17 Mar 2009; 31 Mar 2009; 21 Apr 2009 |
| **Storage conditions** | Store at maximum 8°C | Store at maximum 8°C |

The other two groups were provided with either placebo capsules or capsules containing BB-12^{®} for oral administration (see Table 2).

The label on each aluminum tube contained the following information: 'IMPRESS-capsules with or without probiotics', ' For clinical use only', subject number,' 1 capsule to be taken daily with lunch', 'Contains 30 capsules', expiry date (24 months from date of production), storage conditions, 'To be kept out of reach of children', Sponsor name, Trial code, and Investigator name.

**Table 2 Description of capsules for the IMPRESS-study**

| | **Placebo capsule (Chr. Hansen A/S, Denmark)** | **Probiotic capsule (Chr. Hansen A/S, Denmark)** |
|---|---|---|
| **Brief description** | Capsules with excipients only | Capsules containing BB-12^{®} |
| **Ingredients** | Maltodextrine DE12, Sodium arginate, Silicon dioxide, Magnesium stearate, Microcrystalline Cellulose, Hypromellose (HPMC), Titanium dioxide | Maltodextrine DE12, Sodium arginate, Silicon dioxide, Magnesium stearate, Microcrystalline Cellulose, HPMC, Titanium dioxide, BB-12^{®}, minimum 1 x 10⁹ CFU/capsule |
| **Supplied as** | Alu-tubes containing 30 Capsules | Alu-tubes containing 30 Capsules |
| **Batch number** | H 1276 | H 1276 |
| **Storage conditions** | Store at maximum 8°C | Store at maximum 8°C |

The subjects were instructed by the research personnel of the Sacco Hospital to drink one drink a day or take one capsule a day at lunch time for 6 weeks (42 days). Bottles and aluminum tubes with capsules were coded to maintain blinding. Compliance was based on subject recordings of missing doses in the diary.

The influenza vaccine used in the study was the Fluad^{®} vaccine for the season of 2008/2009 from Novartis Vaccines and Diagnostics, Siena, Italy with an expiry date of June 10^{th} 2009. The strains that were present in the vaccine were:
A/Brisbane/59/2007 (H1N1)-like strain (A/Brisbane/59/2007, IVR-148), A/Brisbane/10/2007/ (H3N2)-like strain (A/Uruguay/716/2007, NYMCX-175C) and the B/Florida/4/2006-like strain (B/Florida/4/2006). The vaccine was stored at 4°C in the hospital pharmacy. At Visit 3, all subjects received an intramuscular injection with 0.5 mL of vaccination.

### Methods of Subject Assignment to the Treatment Groups

A SAS computer program was used to generate permuted block randomization assignment stratified by age and gender with a block size of 6. Age was stratified as 20-39 years and 40-60 years. Randomization lists were provided by Sprim's statistical team to the sponsor to label the capsule products according to a unique study ID system. The drinks were labeled by a blinded study coordinator according to the randomization list prior to dispensing.

The assignment of a randomization number for each subject was based on chronological order for subjects' accrual within each of the age-gender strata.

All products were blinded.

### Selection of Doses in the Study

A dose of minimum 1 x 10⁹ CFU/day of either BB-12^{®} or L. casei 431^{®} was selected for this study as this is the daily dose usually required to modulate the immune system (Minelli and Benini 2008).

### Selection and Timing of Dose for Each Subject

Subjects were instructed by the research personnel to consume the drink or take the capsule at lunch time. Instructions for consumption were also written on the packaging of the study product. Product consumption at lunch time was chosen because, in Italy, it is more common to consume a dairy product at lunch time than in the morning at breakfast.

### Blinding

Appearance, shape, smell and taste were indistinguishable between placebo and probiotic drinks and between placebo and probiotic capsules, respectively. The identity of the specific product (active vs. placebo) was blind to the subjects, the support staff and the investigators. The site pharmacy received a set of sealed envelopes containing the product identity of each randomization number, which could be broken in case of emergency. No code breaks were performed during the study.

### Efficacy Variables

### Efficacy Measurements Assessed and Flow Chart

An overview of the scheduling of study measures and procedures is provided in Table 3.

**Table 3 Study schedule for the IMPRESS-study**

| | **Screening period** | **Supplementation period** | | | **Follow-up period** |
|---|---|---|---|---|---|
| **Visit** | **V1** | **V2** | **V3** | **V4** | **V5** |
| **Day** | **-14** | **0** | **14** | **42** | **112** |
| **Instruction on diet without fermented products/probiotics** | X | X | | | |
| **Inclusion and exclusion criteria** | X | X | | | |
| **Confirmation of elligibility** | | X | | | |
| **Subject medical history** | X | X | | | |
| **Informed consent** | | X | | | |
| **Randomization** | | X | | | |
| **Vaccination** | | | .X | | |
| **Fasting blood sample¹** | | X | | X | |
| **Saliva sampling²** | | X | | X | |
| **Clinical examination³** | | X | X | X | |
| **Phone call to check for influenza symptoms, AEs and concomitant drug use** | | | | | X |
| **Symptom diary** | | Every week | | | |
| **Influenza-like-illness evaluation in diary** | | Every 4 weeks based on the weekly Symptom Diary | | | |
| **Adverse Events** | | X | X | X | X |

| | | | | | |
|---|---|---|---|---|---|
| ¹ for analysis of total IgG, IgA, IgM, IgG1, and IgG3; vaccine-specific IgG, IgG1, and IgG3; IL-2, INF-γ, and IL-10; NK activity, CD4+ T cells, phagocytosis; tetanus-specific IgG ² for analysis of total and vaccine-specific salivary IgA, IgG and IgM ³ at V2 and V4 the clinical examination included height and weight, vital signs (heart rate, blood pressure, temperature and respiratory rate), and infection status; at V3 examination was for infection status only | | | | | |

### Primary Efficacy Variables

### Antigen-Specific Immune Responses

- Plasma levels of vaccine-specific IgG, IgG1 and IgG3
- Levels of vaccine-specific salivary IgG, IgA and IgM

### Secondary Variables

### Response of the Adaptive Immune System

- Plasma level of total IgG, IgA, IgM, IgG1 and IgG3
- Levels of total salivary IgG, IgA and IgM
- Plasma concentrations of cytokines IFN-γ, Il-2 and Il-10

### Response of the Innate Immune System

- NK cell activity
- CD4 positive T lymphocytes
- Phagocytosis and phagocyte killing

### Effects on Upper Respiratory Tract Injections/Symptoms and Influenza-Like Illness

Subjects were provided with a paper diary for the supplementation and follow-up periods, where the subject evaluated symptoms of URTI by completing a symptom diary (McDowell 2006) every week and an ILI evaluation every 4 weeks based on the symptom diary. Diaries were provided in the local language.

### Infection Status

Infection status was evaluated by a clinical examination at Visits 1 to 4. Infection (yes/no) rate was established clinically by assessing presence of fever, flu-like symptoms including throat infection and general malaise and/or body ache. The evaluation of infection status used was developed from the World Health Organization (WHO) criteria of seven symptoms as listed below.

Length of infection was documented by number of days. Infection was defined as a viral infection that affected the nose, throat, and bronchi for approximately 7 days and was characterized by the following:
- Onset of high fever (yes/no)
- Aching muscles (yes/no)
- Severe headache (yes/no)
- Severe malaise (yes/no)
- Nonproductive cough (yes/no)
- Sore throat (yes/no)
- Rhinitis (yes/no)

For symptoms lasting more than 7 days, the particular symptom and the type of treatment administered, if any, were documented.

For each "yes" response to the events listed above, the following details were recorded:
- date of diagnosis
- date of onset of symptoms
- date of symptom cessation
- medications taken
- which symptoms medications were prescribed for

### Handling of Biological Samples and Laboratory Analyses Procedures

An overview of the parameters measured for each study objective and the laboratory method employed is provided in Table 4 below.

**Table 4 Laboratory analyses of efficacy and safety variables for the IMPRESS study**

| **Objectives** | **Parameters** | **Specimen** | **Laboratory analyses** |
|---|---|---|---|
| *Antigen-specific immune response* | | | |
| Vaccine-specific antibodies in plasma | IgG | Plasma | ELISA |
| | IgG1 | | |
| | IgG3 | | |
| | | | |
| Vaccine-specific antibodies in saliva | IgG | Saliva | ELISA |
| | IgA | | |
| | IgM | | |
| *Impact on adaptive immunity* | | | |
| Total antibodies in plasma | IgG | Plasma | ELISA |
| | IgA | | |
| | IgM | | |
| | IgG1 | | |
| | IgG3 | | |
| Total antibodies in saliva | IgA | Saliva | ELISA |
| | IgG | | |
| | IgM | | |
| Plasma circulating cytokines | IL-2 | Plasma | ELISA |
| | IFN-γ | | |
| | IL-10 | | |
| *Impact on innate immunity* | | | |
| | NK cell activity | PBMCs | Fluorescence-activated cell sorter (FACS) and Flow cytometry |
| | CD4+ T cells | | |
| | Phagocytosis and | | |
| | phagocyte killing | | |
| *Overstimulation or dysregulation of homeostasis* | | | |
| Autoantibodies in plasma | Tetanus-specific IgG | Plasma | ELISA |

### EXAMPLE 3

### Handling of Biological Samples

### Blood Sampling

Fasting blood samples were collected at Visits 2 and 4 by venapuncture in the antecubital vein in 3 vacutainer tubes (7 mL) containing EDTA (Becton Dickinson & Co., Rutherford, NJ) by a member of the research team. Blood samples were stored ambiently for up to 2 hours before centrifugation.

### Plasma Collection

Plasma samples were obtained by centrifugation of non-coagulating whole blood (EDTA) for 10 min at 1400 rpm and 20 °C. After centrifugation, 2 mL of plasma was transferred into 2 cryotubes per subject and immediately frozen at -80°C until analyses.

### Peripheral Blood Mononuclear Cell (PBMC) Separation

After plasma collection PBMCs were separated from the buffy coat on lymphocyte separation medium (Organon Teknica Corp., Durham, NC), washed twice in phosphate buffered saline (PBS) (Organon Teknica) and centrifuged at 1900 rpm for 10 minutes. Working on ice, 1 mL of a freezing solution (85% FBS-supplemented RPMI + 15% DMSO) was added to the PBMC pellet and cells were re-suspended. Finally, the cell suspension was transferred to 2 mL cryovials and frozen at -80°C until use at a quantity of 10-15x10⁶ viable PBMCs per vial (as determined by trypan blue exclusion).

### Saliva sampling

Saliva was sampled at Visits 2 and 4 according to the spitting method. The subject was in an upright position and asked to spit out the saliva that was accumulated in the floor of the mouth into a sterile test tube. Subjects spit out the saliva every 60 seconds for 5 minutes, and the tubes containing the saliva were immediately frozen at -80°C until analyses. Saliva samples were collected after an overnight fast.

### EXAMPLE 4

### Methods of Laboratory Analyses

All laboratory samples were analyzed in duplicate using commercial kits, and according to the short descriptions below.

### Vaccine-Specific Antibodies in Plasma

Vaccine-specific IgG in plasma were analyzed using ELISA technique according to the manufacturer's instructions provided with the Influenza A IgG ELISA kit (IBL-America, Inc., MN, USA). The microtiter plates provided with these assays had been pre-coated with influenza A antigen, and binding between IgG antibodies in the samples and the immobilized antigen was detected by a secondary enzyme conjugated antibody specific for human IgG. The addition of a substrate (tetramethylbenzidine, TMB) induced development of a blue color. After the substrate reaction, a Stop Solution was added, which changed the color to yellow, and optical density (OD) was measured within 60 minutes with a spectrophotometer at 450 nm. The intensity of the color developed is directly proportional to the amount of IgG-specific antibodies in the samples. The amount of IgG in the samples was determined by using the standard curve. The detection limit of Influenza A IgG in this assay was 1.09 U/mL.

Vaccine-specific IgG1 and IgG3 in plasma were analyzed using ELISA techniques according to the manufacturer's instructions provided with the Influenza A IgG ELISA kit (IBL-America, Inc., MN, USA) described above, and with the following modification: the specific antibodies in the samples, which were bound to the immobilized antigen, were detected by a secondary Horse Radish Peroxidase (HRP)-conjugated antibody specific for human IgG1 or IgG3 (Alpha Diagnostic Intl. Inc., Texas, USA) with a dilution of 1:2000. After the substrate reaction, a Stop Solution was added, and OD measured within 60 minutes with a spectrophotometer at 450 nm. The intensity of the color developed is directly proportional to the amount of IgG-specific antibodies detected. The amount of IgG1 or IgG3 in the samples was determined directly using the standard curve. The detection limit of Influenza A IgG in this assay was 1.09 U/mL.

### Vaccine-Specific Antibodies in Saliva

Vaccine-specific antibodies IgG, IgA and IgM in saliva were analyzed using ELISA techniques according to the manufacturer's instructions provided with the Influenza A IgG/IgA/IgM ELISA kits (IBL-America, Inc., MN, USA). The microtiter plates provided with these assays had been pre-coated with Influenza A antigen. Specific antibodies in the samples, which were binding to the immobilized antigen, were detected by a secondary enzyme conjugated antibody specific for human IgG, IgA or IgM. After the substrate reaction; OD was measured with a spectrophotometer at 450 nm within 60 min after pipetting of the Stop Solution. The intensity of the color developed is proportional to the amount of IgG, IgA or IgM-specific antibodies detected. The amount of specific antibodies in each sample was determined directly using the standard curve. The detection limit of Influenza A IgG, IgA and IgM in these assays were 1.09 U/mL, 1.29 U/mL and 1.17 U/mL, respectively.

### Tetanus-Specific IgG in Plasma

Tetanus-specific IgG in plasma were analyzed using the ELISA techniques according to the manufacturer's instructions provided with the tetanus Antibody Elisa Kit (Wuhan Institute of Biologic Product, Wuhan, China). The microtiter plate provided in this kit had been pre-coated with an antibody specific to tetanus-specific IgG. Standards or samples were then added to the appropriate microtiter plate wells. A biotin-conjugated polyclonal antibody preparation specific for tetanus-specific IgG and Avidin conjugated to HRP was also added to each microplate well and incubated. Then a TMB substrate solution was added to each well, and wells that contained tetanus-specific IgG, biotin-conjugated antibody and enzyme-conjugated Avidin exhibited a change in color. The enzyme-substrate reaction was terminated by the addition of a sulphuric acid solution and the color change was measured spectrophotometrically at a wavelength of 450 nm. The concentration of tetanus-specific IgG in the samples was determined by comparing the OD of the samples to the standard curve. The detection range of tetanus-specific IgG in this assay was 0.1-40 IU/L, and the lower limit of detection was 0.1 IU/L.

### Total Antibodies in Plasma

Total concentration of plasma IgA, IgG, IgM, IgG1 and IgG3 were analyzed using the ELISA technique according to the manufacturer's instructions provided with the Human Immunoglobulin ELISA kits (Groundwork Biotechnology Diagnosticate, San Diego, USA). For these antibodies using the ELISA method, the stop solution changed the color from blue to yellow and the intensity of the color was measured at 450 nm using a spectrophotometer. In order to measure the concentration of antibody in the sample, each ELISA Kit included a set of calibration standards. The calibration standards were assayed in duplicate at the same time as the samples and resulted in a standard curve of OD versus antibody concentration. The concentration of antibody in the samples was then determined by comparing the OD of the samples to the standard curve. The detection limit for IgG1 and IgG3 was 0.01 mg/mL. The sensitivity of IgG, IgA and IgM assays were 1.0mg/mL, 0.01mg/mL, and 0.1mg/mL, respectively.

### Total Antibodies in Saliva

Total concentration of salivary SIgA was analyzed using the ELISA technique and according to the manufacturer's instructions provided with the Human secretory immunoglobulin A, SIgA ELISA kit (USCNLIFE™ Wuhan, CHINA). The microtiter plate provided in this kit had been pre-coated with an antibody specific to SIgA. Standards or samples were then added to the appropriate microtiter plate wells. A biotin-conjugated polyclonal antibody preparation specific for SIgA and Avidin conjugated to HRP was also added to each microplate well and incubated. Then a TMB substrate solution was added to each well, and wells that contained SIgA, biotin-conjugated antibody and enzyme-conjugated Avidin exhibited a change in color. The enzyme-substrate reaction was terminated by the addition of a sulphuric acid solution and the color change was measured spectrophotometrically at a wavelength of 450 nm. The concentration of SIgA in the samples was determined by comparing the OD of the samples to the standard curve. The detection limit of SIgA in this assay is typically 3.9 ng/mL and the lower limit of detection was defined as the lowest protein concentration that could be differentiated from zero.

Total concentration of salivary IgG or IgM was analyzed using ELISA techniques according to the manufacturer's instructions provided with the Quantitative Human IgG ELISA/Quantitative Human IgM ELISA kits (ZeptoMetrix Corporation, NY, USA). The concentration of the samples was adjusted to obtain the optimal detection concentration. The microtiter plates provided with these assays had been pre-coated with polyclonal antibodies to human IgG or IgM. Standards or samples were added in duplicate to the appropriate microtiter plate wells and incubated. The detector antibody conjugated with HRP was pipetted into each standard and sample well. Following incubation, a TMB substrate solution was added to each well and a blue color developed in wells containing human IgG or IgM. The enzyme-substrate reaction was terminated by the addition of a sulphuric acid solution, and a color change from blue to yellow occurred. The color change was measured spectrophotometrically at a wavelength of 450 nm. The concentration of IgG or IgM in the samples was determined by comparing the OD of the samples to the standard curve. The detection limit of IgG and IgM in these assays is typically 3.9 ng/mL, and the lowest limit of detection was defined as the lowest protein concentration that could be differentiated from zero.

### Il-2, INF-γ and IL-10 in Plasma

Plasma concentration of cytokines was analyzed using the ELISA techniques according to the manufacturer's instructions provided with the Human IL-2/INF-γ /IL-10 Immunoassay kits (Quantikine^{®}, R&D Systems, Inc., MN, USA). The microtiter plates provided with these assays had been pre-coated with a monoclonal antibody specific for IL-2, IFN-γ or IL-10. Standards and samples were pipetted into the wells in duplicate and the present cytokine was bound by the immobilized antibody. After washing away any unbound substances, an enzyme-linked polyclonal antibody specific for IL-2, IFN-γ or IL-10 was added to the wells. Following a wash to remove any unbound antibody-enzyme reagent, a substrate solution was added to the wells and color developed in proportion to the amount of cytokine bound in the initial step. The color development was stopped and the intensity of the color was measured by a spectrophotometer at a wavelength of 450 nm. The concentration of cytokine in the samples was determined by comparing the OD of the samples to the standard curve. The detection limits for IL-2, INF-γ and IL-10 in these assays are typically less than 7.0, 8.0 and 3.9 pg/mL, respectively,

### Phagocytosis and Phagocyte Killing

Phagocytosis and killing was determined according to the method previously described by Saresella and co-workers (1997). Whole blood leukocytes (CD13+ cells) were incubated with opsonized fluorescein isothiocyanate-labeled (FITC-labeled) C. Albicans blastospores for phagocytosis and killing assays. Only live FITC-labeled C. *albicans* blastospores were used as controls.

Phagocytosis and killing was determined by gating the phagocytes (CD13+ cells) and calculating the percentage of phagocyte-associated green fluorescent cells. This procedure is based on the observation that FITC-labeled *C. albicans* blastospores lose their green fluorescence and acquire red fluorescence after staining with Ethidium bromide (EtBr). Therefore, internalized *C. albicans* blastospores remain green, whereas adherent and non-phagocytosed blastospores stain red. The percentage of phagocytosing and killing PMN was equal to the number of green- and double-labeled green and red.

The cytometric analysis of phagocytosis and killing was performed using a Coulter EPICS XL Flow Cytometry, equipped with an air-cooled 15 mW argon ion laser operating at 488 nm. Multiparametric data were collected based on registration of approximately 10,000 events and analyzed using Coulter System II software. Green fluorescence from FITC was measured by means of a 525 nm band pass filter, while red fluorescence from EtBr was measured through a 620 nm band pass filter.

Four different groups were identified: Phagocytes with adherent FITC-labeled C. Albicans blastospores (% adherence), phagocytes with ingested and adherent FITC-labeled C. Albicans blastospores (% adherence and ingestion), phagocytes with ingested FITC-labeled C. Albicans blastospores only (% ingestion), and phagocytes with no interaction (% no interaction).

### Natural Killer Cell Activity

Natural Killer (NK) cell assessment began by filling a 50 mL test tube with 50 mL prewarmed (37°C) complete medium. One vial of K562 target cells was rapidly thawed by rapid agitation in 37°C water bath. When the ice was melted, the vial was removed from the water bath, the cell suspension was transferred to the tube containing warm complete medium and mixed gently. The cell suspension was centrifuged at 1500 rpm for 5 min at room temperature. The supernatant was discarded and the cell pellet was re-suspended in 1 mL of complete medium. Cell number was determined by trypan blue exclusion test and cell concentration was adjusted to 1x10⁵/mL in complete medium. Effector cells were thawed and prepared as the target cells. The cell number was determined by trypan blue exclusion test and cell concentration was adjusted to 1x10⁵/mL in complete medium. For "high control" samples 30 µl IL-2 (200 U/mL) was added to the effector cell suspension. Effector cells were mixed with K562 target cells at a 25:1 E:T ratio in a final volume of 200 µl. Target cells alone were used as control. All tubes were vortexed and centrifuged for 3 min at 1500 rpm. Tubes were then incubated for 120 min in a humified CO₂ incubator. 50 µl DNA staining solution was added per tube, vortexed and incubated for 5 min on ice. Finally, 250 µl PBS was added and samples analyzed by flow cytometry. NK cell activity was calculated as % target cells killed at an E:T ratio of 25:1.

### CD4 Positive T Lymphocytes

CD4+ T cells were analyzed with flow-cytometric techniques using a monoclonal antibody (Phycoerythrin anti-human CD4; eBioscience™, San Diego, USA) that reacts with human CD4, a 59kDa cell surface receptor expressed by a majority of thymocytes, a subpopulation of mature T helper cells, and, in low levels, monocytes. This antibody was pre-titrated and tested by flow cytometric analysis of normal human PBMCs. This was used at 5 µl (0.125 µg) per million cells in a 100 µl total staining volume.

### EXAMPLE 5

Statistical analyses were performed using the SAS package version 9.2 (SAS Institute Inc., Cary, NC, USA).

### Multiple Comparisons (MC)

The primary efficacy variables were adjusted for multiplicity by the Holm-Bonferroni method (Holm 1979). Only related pairs were compared statistically: L. casei 431^{®} drink was compared with placebo drink and BB-12^{®} capsule was compared with placebo capsule, giving two comparisons for each parameter. There were six primary efficacy endpoints (specific IgG, IgG1 and IgG3 in plasma and specific IgA, IgG and IgM in saliva) adding the number of comparisons to 12 for the primary efficacy evaluation.

In the Holm-Bonferroni method all 12 comparisons were first performed unadjusted and the p-values were ordered from smallest to largest. The smallest p-value was compared to an adjusted level of significance equal to alphaa_{adj}= 5%/12 (alpha/number of comparisons) = 0.004. If the smallest p-value was higher than alpha_{adj} it was concluded that all comparisons were non-significant. If the smallest p-value was below alpha_{adj} the corresponding comparison was concluded to be statistically significant at a level of 5%. At the next step the second lowest p-value was compared with alpha_{adj} =5%/11 using the same method as described above. This process was continued until a p-value above alpha_{adj} was found, and the remaining comparisons were concluded to be non-significant.

The statistical analyses for secondary objective endpoints were not adjusted for multiple testing.

### Analysis of Efficacy

### Analyses of Primary Objectives

The primary efficacy variable analyzed statistically was the change from baseline i.e. the difference between Day 42 and Day 0 assessments. Univariate analysis of variance was used to identify study effects.

Statistical analyses between groups were also performed within Baseline, within Day 42 and for the Mean Fold Increase (MFI). MFI was defined as (Day 42-Day 0)/Day 0.

Additionally, an increase in specific antibodies of at least two-fold from Baseline to Day 42 was considered substantial, and defined as Difference (Day 42-Day 0) ≥ 2 x Day 0 (Stephanova et al. 2002, Kurstak 1985), and the number of subjects in each group with a substantial increase in vaccine-specific antibodies was calculated.

Only groups within the same product were compared, i.e. L. casei 431^{®} drink vs. placebo drink and BB-12^{®} capsule vs. placebo capsule.

All ANOVA models contained terms of treatment, gender, age and baseline except for the analysis within baseline where the model contained terms of treatment, gender and age. As BMI was significantly different between groups, a post-hoc analysis was performed with BMI as an additional covariate in the ANOVA models.

Geometric mean evaluations were calculated and reported descriptively. Geometric means were calculated by taking the anti-log of the arithmetic mean of the logarithm values.

### Analyses of Secondary Objectives

These analyses followed the same methodological strategies as outlined for the primary objectives.

### Summary of the Data and Descriptive Statistics

Continuous variables like demographic and baseline variables as well as immunoglobulin values were summarized by treatment group using mean, standard deviation (SD), median, 95% Confidence Interval (CI), 5% and 95%-quantiles, and minimum and maximum values. Summaries for categorical variables like gender and proportion of subjects achieving a substantial increase in their antibody values relied on counts and frequencies within each treatment group.

### Distributional Assumptions Verifications

For continuous outcome measures, the focus was on determining normality of the data (Gaussian distribution). This was assessed using Wilk and Shapiro (1965) technique as well as QQ plots.

For some parameters the normal distribution assumption was violated. In these cases, non-parametric methods, i.e. Mann-Whitney Test was also performed as an alternative analysis. All results of these alternative analyses were in line with the results from the parametric analyses.

### Validation of the Randomization Scheme

The goal of these analyses were to determine if significant differences (imbalances) existed among the study groups in terms of "known" predictive covariates like age, gender and baseline immunoglobulin values. These analyses relied on ANOVA or non-parametric tests to evaluate equality of mean values within related groups (i.e. *L. casei* 431^{®} drink vs. placebo drink and BB-12^{®} capsule vs. placebo capsule).

### Data Sets Analyzed

Overall, data were included for 211 subjects in the ITT analysis set, and for 196 subjects in the PP analysis set.

Only results from the ITT analyses are reported here.

### Demographic and Other Baseline Characteristics

In Table the demographic and baseline characteristics of the subjects in the ITT analysis set are shown. The overall mean (SD) age was 33.2 (13.1) years and ranged from 19 to 60 years. Overall, there were more females than males: 118 (55.9%) females, and 93 (44.1%) males.

**Table 5 Demographic and baseline characteristics of the subjects of the IMPRESS- study (ITT analysis set)**

| | **Placebo drink n=54** | **L. casei 431^{®} Drink n=56** | **Placebo capsule n=48** | **BB-12^{®} capsule n=53** |
|---|---|---|---|---|
| Sex (n, %) | | | | |
| Male | 19 (35.2%) | 25 (44.6%) | 21 (43.8%) | 28 (52.8%) |
| Female | 35 (64.8%) | 31 (55.4%) | 27 (56.3%) | 25 (47.2%) |
| Age (years) | | | | |
| Mean (SD) | 35.1 (14.3) | 37.3 (13.9) | 30.9(11.2) | 29.0(11.2) |
| (range) | (20-58) | (20-60) | (19-52) | (19-55) |
| Race (n, %) | | | | |
| | White | White | White | White |
| | 54 (100%) | 56 (100%) | 48 (100%) | 53 (100%) |
| BMI (kg/m²) | | | | |
| Mean (SD) | 22.8 (3.6) | 24.6 (4.3) * | 22.4 (3.8) | 22.8 (4.1) |
| (range) | (17-33) | (17-39) | (17-34) | (17-37) |
| Resp/Min | | | | |
| Mean (SD) | 16 (1) | 15 (1) * | 16(2) | 16 (1) |
| (range) | (12-20) | (12-17) | (12-24) | (12-18) |
| Heart rate (BPM) | | | | |
| Mean (SD) | 69 (7) | 70 (7) | 69(7) | 68 (6) |
| (range) | (56-84) | (50-90) | (54-84) | (52-87) |
| Temperature (°C) | | | | |
| Mean (SD) | 36.1 (0.5) | 36.1 (0.5) | 36.1 (0.4) | 36.1 (0.4) |
| (range) | (35.0-37.2) | (35.1-37.1) | (35.2-36.8) | (35.0-36.8) |
| Systolic Blood pressure | | | | |
| Mean (SD) | 112(7) | 116 (8) * | 113 (11) | 112(8) |
| (range) | (100-130) | (100-130) | (85-140) | (100-135) |
| Diastolic Blood pressure | | | | |
| Mean (SD) | 74 (7) | 78 (7) * | 74 (8) | 74 (8) |
| (range) | (60-90) | (60-95) | (60-95) | (50-95) |

| | | | | |
|---|---|---|---|---|
| * p<0.05 for difference vs. placebo drink group, analyzed with Kruskal-Wallis test | | | | |

### Efficacy Results

### Primary Objectives

### Vaccine-specific IgG, IgG1, and IgG3 in Plasma

Significant differences between probiotic and placebo groups were demonstrated for all three parameters in the primary analysis of change from baseline. When comparing the differences between Day 42 and Baseline, the vaccine-specific antibody values in the BB-12^{®} group were significantly more increased from Baseline to Day 42 than in the placebo group (p<0.001 for all parameters). Likewise, the specific antibody values in the *L. casei* 431^{®} group were significantly more increased than in the corresponding placebo group (p=0.010 for IgG and p<0.001 for IgG1 and IgG3).

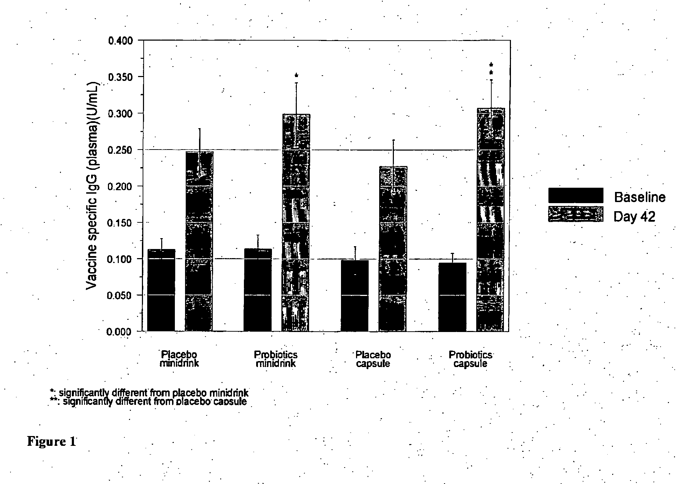

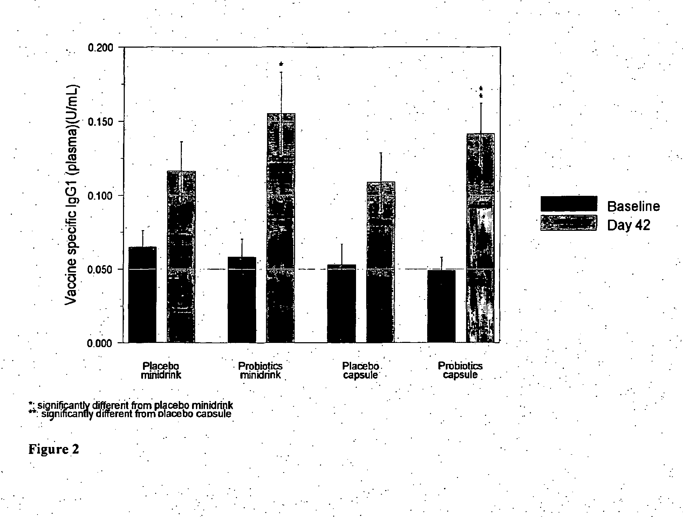

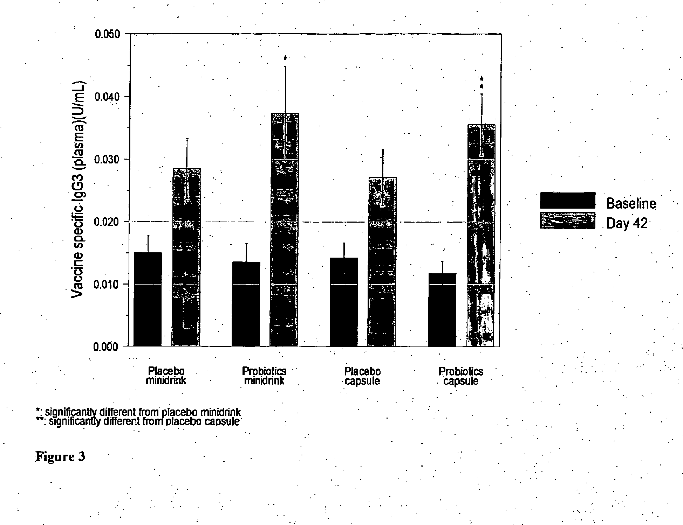

Additional analyses showed that Baseline values of vaccine-specific IgG, IgG1, and IgG3 were similar across all study groups. Cross-sectional analyses of Day 42 values showed for all three parameters significantly higher values for the BB-12^{®} group vs. the placebo group and for the L. casei 431^{®} group vs. the placebo group (p<0.001 for all three parameters) (Figure 1, Figure 2 and Figure 3.

Influenza vaccine-specific values for IgG, IgG1 and IgG3 were also evaluated for Mean-Fold Increase (MFI). Significant group differences in MFI were observed for the BB-12^{®} group vs. placebo group for IgG (p=0.016), and for the BB-12^{®} group vs. placebo group and L. casei 431^{®} group vs. placebo group for IgG1 and IgG3 (p<0.001 for all) (Table 6).

**Table 6 Mean Fold Increase of Influenza Vaccine-Specific Plasma IgG, IgG1 and IgG3 by Study Group**

| **Variable** | **Placebo Drink (n=54)** | **L. casei 431^{®} Drink (n=56)** | **Placebo Capsule (n=48)** | **BB-12^{®} Capsule (n=53)** |
|---|---|---|---|---|
| *Specific IgG* | | | | |
| Mean (SD) | 1.62 (1.91) | 1.81 (0.53) | 1.68 (2.05) | 2.36 (0.48) |
| Median | 0.91 | 1.84 | 1.15 | 2.40 |
| 95% CI | 1.10 - 2.15 | 1.67 - 1.95 | 1.08 - 2.27 | 2.23 - 2.49 |
| p-value* | 0.36 | | 0.016 | |
| *Specific IgG1* | | | | |
| Mean (SD) | 0.85 (0.47) | 1.86 (0.49) | 1.27 (0.57) | 2.10 (0.60) |
| Median | 0.78 | 1.92 | 1.17 | 2.02 |
| 95% CI | 0.73 - 0.98 | 1.73 - 1.99 | 1.10 - 1.43 | 1.94 - 2.27 |
| p-value* | <0.001 | | <0.001 | |
| *Specific IgG3* | | | | |
| Mean (SD) | 0.99 (0.29) | 1.99 (0.66) | 0.97(0.33) | 2.21 (1.07) |
| Median | 0.96 | 1.88 | 0.95 | 2.13 |
| 95% CI | 0.91 - 1.07 | 1.82 - 2.17 | 0.87 - 1.07 | 1.91 - 2.50 |
| p-value* | <0.001 | | <0.001 | |

| | | | | |
|---|---|---|---|---|
| *: for difference between groups in MFI analyzed with ANOVA using sex, age and baseline as covariates Source: Appendix 16.1.9 tables 3.1, 3.2, 3.3, 8.1, 8.2 and 8.3 | | | | |

A Mean Fold Increase in vaccine-specific IgG, IgG1 and IgG3 of at least 2 fold was defined as a substantial increase, and the proportion of subjects who achieved a substantial increase was calculated for each group. The number of subjects with a substantial increase in specific antibody values was considerably greater in each probiotic group compared to the relevant placebo group (Table 7).

**Table 7 Number of Subjects with Substantial Increase in IgG, IgG1 and IgG3 Values by Study Group**

| **Variable** | **Placebo Drink** (n=54) | **L. casei 431^{®} Drink** (n=56) | **Placebo Capsule** (n=48) | **BB-12^{®} Capsule** (n=53) |
|---|---|---|---|---|
| *Specific IgG* | | | | |
| Substantial increase (yes/no) | 10/44 | 16/40 | 10/38 | 43/10 |
| Substantial increase (% of n) | 18.5 | 28.6 | 20.8 | 81.1 |
| *Specific IgG1* | | | | |
| Substantial increase (yes/no) | 1/53 | 21/35 | 4/44 | 27/26 |
| Substantial increase (% of n) | 1.9 | 37.5 | 8.3 | 50.9 |
| *Specific IgG3* | | | | |
| Substantial increase (yes/no) | 1/53 | 26/30 | 2/46 | 35/18 |
| Substantial increase (% of n) | 1.9 | 46.4 | 4.2 | 66.0 |

### Vaccine-specific IgG, IgA and IgM in Saliva

The primary analysis of difference between groups in change from baseline showed no significant differences for vaccine-specific salivary IgG, IgA or IgM. However, for IgA a trend towards a significant difference was shown between the BB-12^{®} capsule group and the corresponding placebo group (adjusted p=0.084).

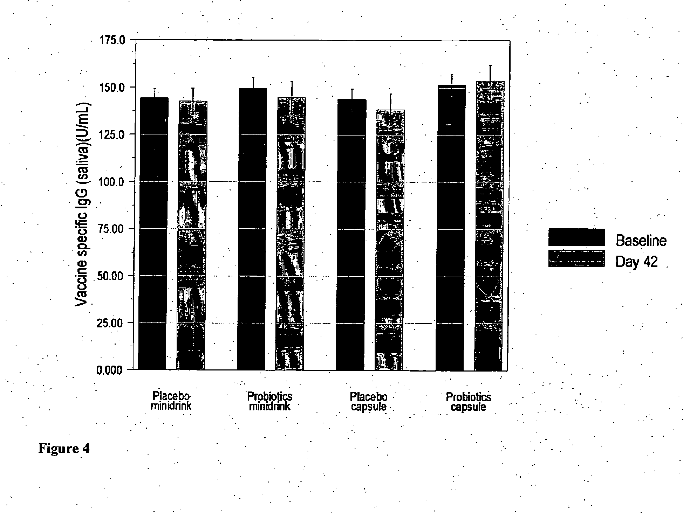

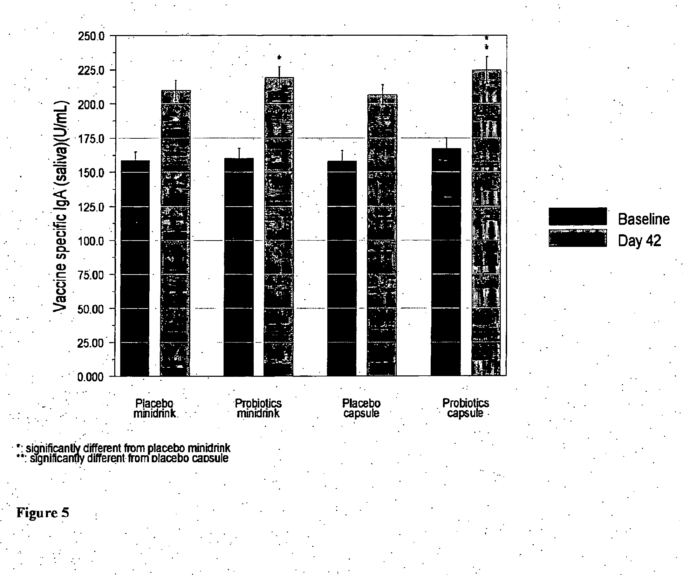

Baseline values of IgG and IgA were similar between groups while the IgM value was slightly higher in the BB-12^{®} group compared to the placebo group at baseline (p=0.007) (Figure 4, Figure 5, and Figure 6)

Cross-sectional analyses of Day 42 values showed higher IgA values in the BB-12^{®} group compared to the placebo group (p=0.014) and in the L. casei 431^{®} group compared to the placebo group (p=0.047) (Figure 4, Figure 5, and Figure 6). Likewise, analysis of Mean Fold Increase revealed a greater MFI in vaccine-specific IgA in each probiotic group compared to the relevant placebo group (Table 8).

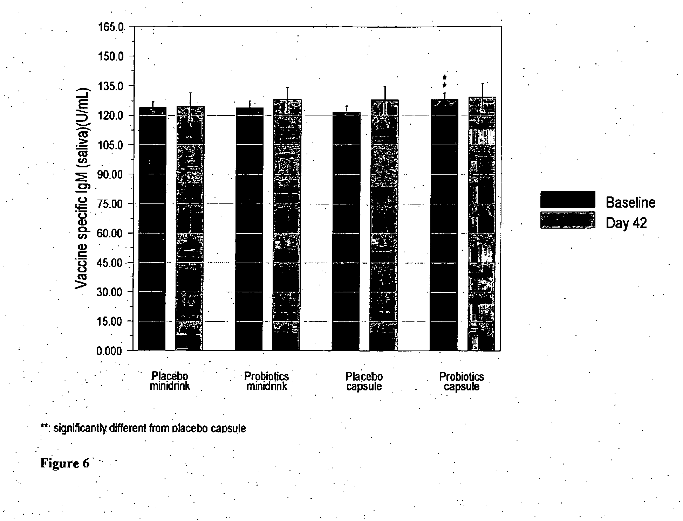

Significant differences between groups were neither seen in IgM or IgG at Day 42 nor in MFI (Figure 4 and Figure 6).

**Table 8 Mean Fold Increase of Influenza Vaccine-Specific Salivary IgA, IgG and IgM by Study Group**

| **Variable** | **Placebo Drink (n=54)** | **L. casei 431^{®} Drink (n=56)** | **Placebo Capsule (n=48)** | **BB-12^{®} Capsule (n=53)** |
|---|---|---|---|---|
| *Specific IgG* | | | | |
| Mean (SD) | -0.02 (0.12) | -0.04 (0.12) | -0.04 (0.13) | 0.01 (0.13) |
| Median | -0.01 | -0.03 | -0.03 | -0.02 |
| 95% CI | -0.05 - 0.02 | -0.07 - -0.00 | -0.08 - 0.00 | -0.03 - 0.05 |
| p-value* | 0.29 | | 0.12 | |
| *Specific IgA* | | | | |
| Mean (SD) | 0.33 (0.14) | 0.39 (0.19) | 0.32 (0.11) | 0.36 (0.21) |
| Median | 0.32 | 0.35 | 0.31 | 0.32 |
| 95% CI | 0.29 - 0.37 | 0.34 - 0.44 | 0.28 - 0.35 | 0.30 - 0.42 |
| p-value* | 0.035 | | 0.017 | |
| *Specific IgM* | | | | |
| Mean (SD) | 0.01 (0.22) | 0.04 (0.19) | 0.06 (0.24) | 0.02 (0.23) |
| Median | -0.03 | 0.02 | 0.04 | -0.06 |
| 95% CI | -0.0 - 0.07 | -0.01 - 0.09 | -0.01 - 0.13 | -0.04 - 0.09 |
| p-value* | 0.47 | | 0.77 | |

| | | | | |
|---|---|---|---|---|
| *: for difference between groups in MFI analyzed with ANOVA using sex, age and baseline as covariates | | | | |

## Claims

1. A probiotic bacterial strain *Bifidobacterium animalis* ssp. *lactis* with the deposit number DSM15954 for use in a method of boosting the immune response in conjunction with an influenza vaccine.

2. A probiotic bacterial strain *Lactobacillus paracasei* ssp. *paracasei* with the deposit number ATCC55544 for use in a method of boosting the immune response in conjunction with an influenza vaccine.

3. The probiotic bacterial strain for use according to claim 1 or 2, wherein the composition is administered at least two weeks prior to vaccination.

4. The probiotic bacterial strain for use according to any one of claims 1 to 3 wherein the concentration of vaccine specific antibodies is significantly increased in treatment groups compared with corresponding placebo treatment groups.

5. The probiotic bacterial strain for use according to claim 4 wherein vaccine specific IgG is increased.

6. The probiotic bacterial strain for use according to claim 5 wherein vaccine specific IgG1 is increased.

7. The probiotic bacterial strain for use according to claim 5 wherein vaccine specific IgG3 is increased.

8. The probiotic bacterial strain for use according to any of claims 1 to 7 wherein the viruses in the influenza vaccine are orthomyxoviruses such as influenza type A, B or C.

9. The probiotic bacterial strain according to any one of claims 1 to 8 wherein the probiotic strain is used in combination with other lactic acid bacteria of other species.

10. The probiotic bacterial strain according to claim 9 wherein the probiotic strain is used in combination with yoghurt ferments.

11. The probiotic bacterial strain according to claim 10 wherein the yoghurt ferments are *Lactobacillus bulgaricus* or *Streptococcus thermophilus.*

12. The probiotic bacterial strain according to any one of claims 1 to 11, wherein the composition is administered during 6 weeks.

13. The probiotic bacterial strain according to any one of claims 1 to 12, wherein the composition is a milk-based drink or a capsule.

## Patentansprüche

1. Probiotischer Bakterienstamm von *Bifidobacterium animalis* ssp. *lactis* mit der Hinterlegungsnummer DSM15954 zur Verwendung in einem Verfahren zur Stärkung der Immunreaktion in Verbindung mit einem Grippeimpfstoff.

2. Probiotischer Bakterienstamm von Lactobacillus paracasei ssp. paracasei mit der Hinterlegungsnummer ATCC55544 zur Verwendung in einem Verfahren zur Stärkung der Immunreaktion in Verbindung mit einem Grippeimpfstoff.

3. Probiotischer Bakterienstamm zur Verwendung nach Anspruch 1 oder 2, wobei die Zusammensetzung mindestens zwei Wochen vor der Impfung verabreicht wird.

4. Probiotischer Bakterienstamm zum Einsatz nach einem beliebigen der Ansprüche 1 bis 3, wobei die Konzentration von impfstoffspezifischen Abwehrstoffen in den Behandlungsgruppen deutlich höher ist als in entsprechenden Placebo-Behandlungsgruppen.

5. Probiotischer Bakterienstamm zur Verwendung nach Anspruch 4, wobei das impfstoffspezifische IgG erhöht ist.

6. Probiotischer Bakterienstamm zur Verwendung nach Anspruch 5, wobei das impfstoffspezifische IgGl erhöht ist.

7. Probiotischer Bakterienstamm zur Verwendung nach Anspruch 5, wobei das impfstoffspezifische IgG3 erhöht ist.

8. Probiotischer Bakterienstamm zum Einsatz nach einem beliebigen der Ansprüche 1 bis 7, wobei die Viren in dem Grippeimpfstoff Orthomyxoviren sind wie zum Beispiel Grippe Typ A, B oder C.

9. Probiotischer Bakterienstamm nach einem beliebigen der Ansprüche 1 bis 8, wobei der probiotische Stamm in Kombination mit anderen Milchsäurebakterien von anderen Arten benutzt wird.

10. Probiotischer Bakterienstamm nach Anspruch 9, wobei der probiotische Stamm in Kombination mit Joghurtfermenten benutzt wird.

11. Probiotischer Bakterienstamm nach Anspruch 10, wobei die Joghurtfermente *Lactobacillus bulgaricus* oder *Streptococcus thermophilus* sind.

12. Probiotischer Bakterienstamm nach einem beliebigen der Ansprüche 1 bis 11, wobei die Zusammensetzung 6 Wochen lang verabreicht wird.

13. Probiotischer Bakterienstamm nach einem beliebigen der Ansprüche 1 bis 12, wobei die Zusammensetzung ein Milchmischgetränk oder eine Kapsel ist.

## Revendications

1. Souche bactérienne probiotique *Bifidobacterium animalis* ssp. *lactis* avec le numéro de dépôt DSM15954 pour l'utilisation dans une méthode pour augmenter la réaction immunitaire conjointement avec un vaccin antigrippal.

2. Souche bactérienne probiotique *Lactobacillus paracasei* ssp. *paracasei* avec le numéro de dépôt ATCC55544 pour l'utilisation dans une méthode pour augmenter la réaction immunitaire conjointement avec un vaccin antigrippal.

3. Souche bactérienne probiotique pour l'utilisation selon la revendication 1 ou 2, où la composition est administrée au moins deux semaines avant la vaccination.

4. Souche bactérienne probiotique pour l'utilisation selon l'une quelconque des revendications 1 à 3 où la concentration des anticorps spécifiques du vaccin est significativement augmentée dans les groupes de traitement par rapport aux groupes de traitement placebo correspondants.

5. Souche bactérienne probiotique pour l'utilisation selon la revendication 4 où la IgG spécifique du vaccin est augmentée.

6. Souche bactérienne probiotique pour l'utilisation selon la revendication 5 où la IgG1 spécifique du vaccin est augmentée.

7. Souche bactérienne probiotique pour l'utilisation selon la revendication 5 où la IgG3 spécifique du vaccin est augmentée.

8. Souche bactérienne probiotique pour l'utilisation selon l'une quelconque des revendications 1 à 7 où les virus dans le vaccin antigrippal sont des orthomyxovirus comme type de grippe A, B ou C.

9. Souche bactérienne probiotique selon l'une quelconque des revendications 1 à 8 où la souche probiotique est utilisée en combinaison avec d'autres bactéries d'acide lactique d'autres espèces.

10. Souche bactérienne probiotique selon la revendication 9 où la souche probiotique est utilisée en combinaison avec des ferments de yaourt.

11. Souche bactérienne probiotique selon la revendication 10 où les ferments de yaourt sont *Lactobacillus bulgaricus* ou *Streptococcus thermophilus.*

12. Souche bactérienne probiotique selon l'une quelconque des revendications 1 à 11, où la composition est administrée pendant 6 semaines.

13. Souche bactérienne probiotique selon l'une quelconque des revendications 1 à 12, où la composition est une boisson à base de lait ou une capsule.
